# EUROPEAN PATENT APPLICATION

(11) **EP 4 115 804 A1**
(43) Date of publication of application: **11.01.2023**
(21) Application number: 22183058.1
(22) Date of filing: 05.07.2022
(51) Int. Cl.: A61B 5/11, A63B 71/08

(54) **DYNAMIC VARIABILITY OF HEAD IMPACT DATA RECORDAL VIA INSTRUMENTED MOUTHGUARD DEVICES**

(30) Priority: 06.07.2021 AU 2021902061
(71) Applicant: HitlQ Limited, South Melbourne, Victoria 3205 (AU)
(72) Inventor: ERIKSON, David, South Melbourne, 3205 (AU); CUEVAS, Luis, South Melbourne, 3205 (AU); VEGAR, Mike, South Melbourne, 3205 (AU); GOODIN, Peter, South Melbourne, 3205 (AU)
(74) Representative: Doherty, William

(57) **Abstract**

The present invention relates, in various embodiments, to technology which facilitates dynamic variability of head impact data Recordal, for example in the context of instrumented mouthguard devices. Some embodiments have been developed to facilitate an event recoding protocol which dynamically adjusts event recoding parameters thereby to provide appropriate data for both "short" and "prolonged" impact events. For example, various embodiments include methods for recording impact events in respect of an instrumented mouthguard device, such methods including dynamically adjusting a period of time for which event data is recorded for a given event responsive to length of time for which an over-threshold condition persists.

## Description

### FIELD OF THE INVENTION

The present invention relates, in various embodiments, to technology which facilitates dynamic variability of head impact data recordal, for example in the context of instrumented mouthguard devices. Some embodiments have been developed to facilitate an event recoding protocol which dynamically adjusts event recoding parameters thereby to provide appropriate data for both "short" and "prolonged" impact events. While some embodiments will be described herein with particular reference to those applications, it will be appreciated that the invention is not limited to such a field of use, and is applicable in broader contexts.

### BACKGROUND

Any discussion of the background art throughout the specification should in no way be considered as an admission that such art is widely known or forms part of common general knowledge in the field.

Brain injuries, particularly those sustained during participation in contact sports, are becoming an increasingly important focus of attention. For example, head impacts sustained during sport can have serious effects of both short term and long-term participant welfare. For example, it is valuable to better understand the nature of a suspected brain injury in terms of: (i) whether a participant should be rested from participation; (ii) an extent to which the injury should prevent a return to activity; (iii) a degree of seriousness of an injury, for instance insofar as that might affect treatment and management; and (iv) better understanding cumulative effects of successive brain injuries for a given participant.

With this in mind, various forms of head impact sensors have been developed, including sensor-fitted helmets, and instrumented mouthguards. There are technical challenges associated with processing data from sensors at those devices thereby to provide for meaningful assessments of head impacts.

### SUMMARY OF THE INVENTION

It is an object of the present invention to overcome or ameliorate at least one of the disadvantages of the prior art, or to provide a useful alternative.

Example embodiments are described below in the section entitled "claims".

One embodiment provides a method for recording events detected by an instrumented mouthguard device, the method including:
(A) collecting data from a plurality of instrumented sensor components provided by the instrumented mouthguard device;
(B) processing at least a subset of the data collected at (A) thereby to identify presence of an over-threshold condition;
(C) in the case that an over-threshold condition is identified:
   (i) commencing recording of event data from a start point preceding presence of the over-threshold condition by a predefined leading period; and
   (ii) determining a point in time where an over-threshold condition is no longer detected; and
   (iii) ending recording of the event data at an end point following the point in time where an over-threshold condition is no longer detected by a predefined trailing period;

such that, for each event, a duration of time for which event data is recorded is dynamically set responsive to the length of time for which the identified over-threshold condition persists.

One embodiment provides an instrumented mouthguard device including:
a mouthguard body configured to be inserted into the mouth of a user;
plurality of instrumented sensor components embedded in the body;
a processing unit embedded in the body; and
a memory module embedded in the body;
wherein the processing unit is configured to receive data from the plurality of instrumented sensor components, and process that data thereby to cause recording discrete sets of event data in the memory module based on an event recording protocol, wherein the event recording protocol includes:
   (i) processing at least a subset of the data collected by the instrumented sensor components thereby to identify presence of an over-threshold condition;
   (ii) in the case that an over-threshold condition is identified, commencing recording of event data from a start point preceding presence of the over-threshold condition by a predefined leading period to an end point following the point in time where the over-threshold condition is no longer detected by a predefined trailing period;

One embodiment provides a method of recording impact events in respect of an instrumented mouthguard device, the method including dynamically adjusting a period of time for which event data is recorded for a given event responsive to length of time for which an over-threshold condition persists.

Reference throughout this specification to "one embodiment", "some embodiments" or "an embodiment" means that a particular feature, structure or characteristic described in connection with the embodiment is included in at least one embodiment of the present invention. Thus, appearances of the phrases "in one embodiment", "in some embodiments" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

As used herein, unless otherwise specified the use of the ordinal adjectives "first", "second", "third", etc., to describe a common object, merely indicate that different instances of like objects are being referred to, and are not intended to imply that the objects so described must be in a given sequence, either temporally, spatially, in ranking, or in any other manner.

In the claims below and the description herein, any one of the terms comprising, comprised of or which comprises is an open term that means including at least the elements/features that follow, but not excluding others. Thus, the term comprising, when used in the claims, should not be interpreted as being limitative to the means or elements or steps listed thereafter. For example, the scope of the expression a device comprising A and B should not be limited to devices consisting only of elements A and B. Any one of the terms including or which includes or that includes as used herein is also an open term that also means including at least the elements/features that follow the term, but not excluding others. Thus, including is synonymous with and means comprising.

As used herein, the term "exemplary" is used in the sense of providing examples, as opposed to indicating quality. That is, an "exemplary embodiment" is an embodiment provided as an example, as opposed to necessarily being an embodiment of exemplary quality.

### BRIEF DESCRIPTION OF THE DRAWINGS

Embodiments of the invention will now be described, by way of example only, with reference to the accompanying drawings in which:
FIG. 1A to FIG. 1D illustrate example configurations of instrumented mouthguard devices.
FIG. 2A TO FIG. 2D illustrate example PCBs component for an instrumented mouthguard device.
FIG. 3 illustrates a technology framework according to one embodiment.
FIG. 4 illustrates an example instrumented mouthguard.
FIG. 5 provides an example of over-threshold condition detection.

### DETAILED DESCRIPTION

The present invention relates, in various embodiments, to technology which facilitates dynamic variability of head impact data Recordal, for example in the context of instrumented mouthguard devices. Some embodiments have been developed to facilitate an event recoding protocol which dynamically adjusts event recoding parameters thereby to provide appropriate data for both "short" and "prolonged" impact events. For example, various embodiments include methods for recording impact events in respect of an instrumented mouthguard device, such methods including dynamically adjusting a period of time for which event data is recorded for a given event responsive to length of time for which an over-threshold condition persists.

The technology described herein relates to processing of data collected by instrumented mouthguard devices. These are devices which are configured to be worn in users' respective mouths, and include a plurality of sensors (for example one or more accelerometers, and optionally at least one gyroscope). Relevant to the present context, a subset of data collected by the sensor devices is recorded as "event data", with each set of event data being intended to describe a respective predicted head impact event. Event data typically includes a set of time series data derived from some or all of the sensor devices (e.g. raw data, processed data, combined data), having a defined start point and a defined end point. Each event has a "duration" defined by an the amount of time between the start point and the end point.

In prior art systems, event data sets are typically defined by a standardised duration (for example 50ms). For example, many known accelerometers and other such sensors are inherently configured to perform event detection, based on an approach whereby a peak acceleration or a threshold acceleration is identified, and an event is recorded using data from a leading and trailing period of predefined duration. In this regard, each event has the same duration, based on the lengths of the predefined leading and trailing periods. The leading and trailing period are in some cases defined by the size of an onboard memory buffer of the sensor.

The present inventors have identified that such an approach, whilst appropriate for observing impacts under in lab conditions, is inadequate in terms of complex real-world scenarios. For example, it has been observed that in certain contact sports a head impact event may continue for a prolonged time, for example due to multiple consecutive impacts (in such cases an impact may be prolonged to the order of 100ms to 1000ms). In such circumstances, a standard duration event Recordal protocol fails to provide an accurate representation of an impact. Accordingly, the present inventors have developed a dynamic recordal expansion approach, whereby a duration of time over which sensor data is recorded for a head impact event is defined by reference to attributes of that event, in particular the duration of an over-threshold condition. In embodiments described below, an event recoding protocol dynamically adjusts a period of time for which event data is recorded for a given event responsive to length of time for which an over-threshold condition persists.

According to one embodiment, a method for recording events detected by an instrumented mouthguard device includes:
(i) Collecting data from a plurality of instrumented sensor components provided by the instrumented mouthguard device. The data is preferably stored in a buffer, for example a buffer of between 10ms and 80ms.
(ii) Processing at least a subset of that data, thereby to identify presence of an over-threshold condition. This over threshold condition may be defined by reference to a range of factors, as discussed in more detail further below.
(iii) In the case that an over-threshold condition is identified, applying a protocol for recording of a set of event data, wherein the duration represented by the set of event data is dynamically defined based on attributes of the over-threshold condition (preferably the duration of time for which the over-threshold condition persists).

The term "over-threshold condition" as used herein is distinguished from a single peak acceleration or the like. The term "over threshold condition" refers to a persistent state, i.e. a condition that persists for a period of time (as opposed to an instant, as is the case in identifying a peak). In particular, an over-threshold condition may persist through multiple peak spikes (such as peak acceleration spikes).

The protocol for recording a set of event data includes commencing recording of event data from a start point preceding presence of the over-threshold condition by a predefined leading period. This leading period is preferably in the order of between 5ms and 30ms. In some embodiments the leading period is dynamically adjusted based on other attributes of the event data (for example a time period for which an over-threshold condition is observed).

The method includes determining a point in time where an over-threshold condition is no longer detected. The protocol ends recording of the event data at an end point following that point in time where an over-threshold condition is no longer detected, specifically following that point by a predefined trailing period. This trailing period is preferably in the order of between 5ms and 30ms. In some embodiments the trailing period is dynamically adjusted based on other attributes of the event data (for example a time period for which an over-threshold condition is observed).

The "predefined" leading and trailing periods may be defined in terms of respective durations, or by reference to an equation or equations which calculate such durations based on other data.

The over-threshold condition may be defined by reference to any one or more of the following:
- Identification of linear acceleration at one of the sensors which exceeds a defined threshold (for example a threshold set at a value between 5G and 20G). This may be defined relative to the magnitude of the acceleration vector in the case of a 3-axis accelerometer. In some cases, rather than using the magnitude of the acceleration vector, an adjusted vector may be defined by applying a weighting protocol to each of the X, Y and Z components.
- Identification of linear acceleration at two or more of the sensors which exceeds a defined threshold (for example a threshold set at a value between 5G and 20G). This may be defined relative to the magnitude of the acceleration vector in the case of a 3-axis accelerometer. In some cases, rather than using the magnitude of the acceleration vector, an adjusted vector may be defined by applying a weighting protocol to each of the X, Y and Z components.
- Identification of rotational acceleration at one of the sensors which exceeds a defined threshold (for example a threshold set at a value between 500 radians per second squared and 5000 radians per second squared).
- Identification of rotational acceleration at two or more of the sensors which exceeds a defined threshold (for example a threshold set at a value between 5G and 20G). This may be defined relative to the magnitude of the acceleration vector in the case of a 3-axis accelerometer. In some cases, rather than using the magnitude of the acceleration vector, an adjusted vector may be defined by applying a weighting protocol to each of the X, Y and Z components.
- Identification of rotational velocity at one of the sensors (e.g. a gyroscope) which exceeds a defined threshold (for example a threshold set at a value between 3 radians per second and 8 radians per second).
- Furthermore, the threshold may be dynamically modified during the impact to have a second (or more) threshold(s) or criteria for determining whether to continue the recording of data. For example, there may be a first threshold defining onset of an over-threshold condition, and one or more subsequent threshold values which need to be met for the over-threshold condition to persist.
- Combinations of the preceding examples.

By such an approach, event recording is dynamically adjusted to account for more complex forms of head impacts, which may occur in activities such as contact sports. This includes impacts where there are multiple acceleration peaks during a short period of time, potentially representing a "prolonged impact" event.

Preferably the event recording protocol is coded into software instructions that execute via a processing unit embedded in the mouthguard device. For example, data from the various sensors is sent to the processing unit, and buffered. A subset of that data (for example linear acceleration from a single sensor) is processed via code representative of the event recording protocol, thereby to define a start and end point for an over-threshold condition, and trigger permanent storage of event data in a memory module (the term "permanent" is used here to differentiate from storage in a temporary buffer). In some embodiments a programmable buffer length is set by hardware, and a variable recoding length set by software.

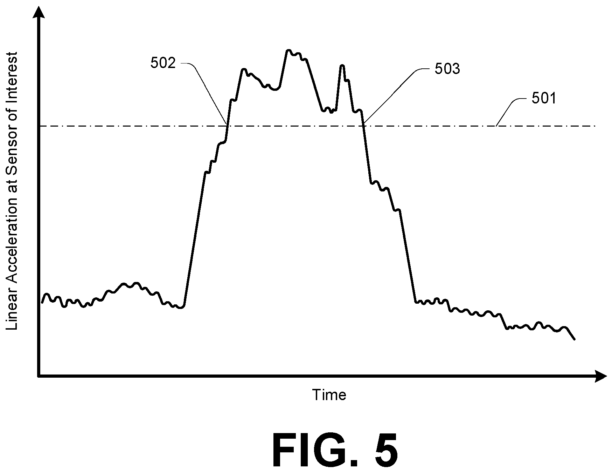

FIG. 5 provides an example of time series data which might be derived from an accelerometer, thereby to provide additional context to the present event recording protocol. In this example, an over-threshold condition limit is set as per line 501, which relates to linear acceleration vector magnitude at a particular three-axis accelerometer embedded in an instrumented mouthguard. The time-series acceleration data is above line 501 from point 502 to point 503. As such, the event recording protocol causes recording of an invent in mouthguard sensor data from: (i) a time preceding point 502 by a predefined leading period; to: (ii) a time following point 503 by a predefined trailing period.

Examples of mouthguards and instrumented components will now be described by reference to the drawings. It will be appreciated that further modifications are possible within the spirit and scope of the present disclosure. Similar features have been designated with corresponding reference numerals.

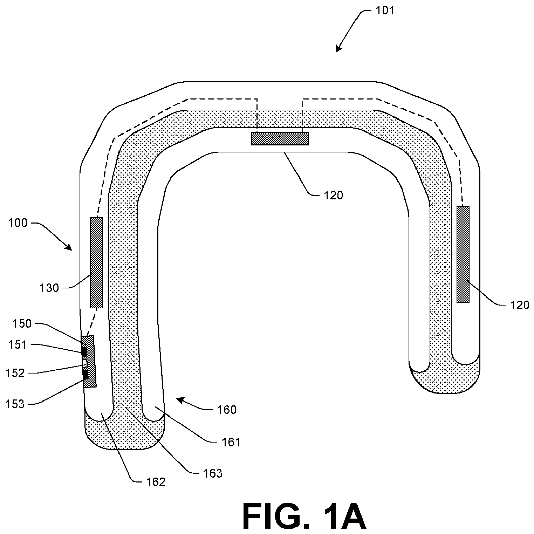

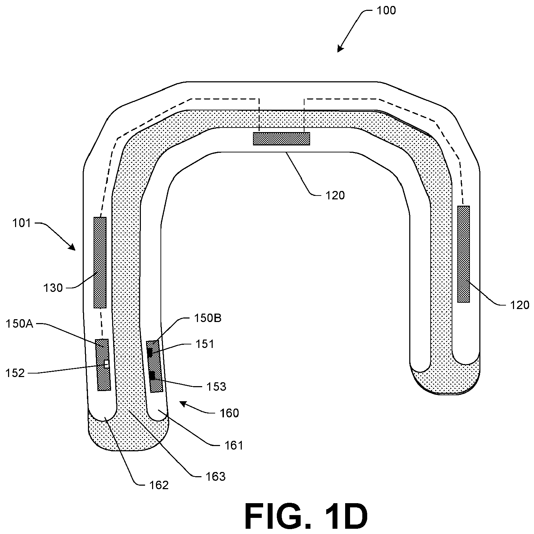

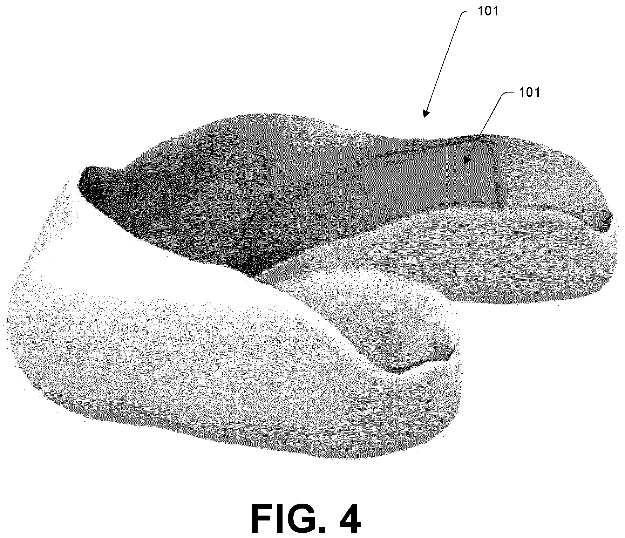

The examples of FIG. 1A to FIG. 1D provide a simplified plan illustration of a mouthguard. These are intended to provide a rough overview of component positioning, and an image showing an example mouthguard in three dimensions is provided in FIG. 4.

In each example, the mouthguard comprises a mouthguard body 100, in which an instrumented component 101 is embedded. The instrumented component is a single printed circuit board with multiple component zones formed thereon; in other examples there are multiple distinct interconnected circuit boards.

In the present embodiment the mouthguard body is custom formed from an inner later that is moulded for a user based on a dentition scanning process, such that the mouthguard inner body provides a customised specifically to that user. The instrumented component 101 is then affixed to the inner body, and an outer body sealed to the inner body 100 thereby to sandwich the instrumented component.

Additional detail regarding example instrumented mouthguard construction processes are provided in Australian provisional patent application 2020904214, entitled "multi-layered instrumented mouthguard devices, and methods for manufacturing of instrumented mouthguard devices". The disclosure of that application is hereby incorporated by cross reference.

Instrumented component 101 includes a plurality of component zones 110, 120 and 130, which are spaced apart on a flexible PCB which follows a meandering path (i.e. the distance between component zones along the PCB is greater than the direct distance between the component zones). The interconnecting PCB member is illustrated as a dashed line in FIG. 1A to FIG. 1D for the sake of simplicity; example circuit boards are described in more detail further below.

The meandering path allows for mounting of the flexible circuit board substrate to the mouthguard inner body, such that the component zones are located in a frontal region of the mouthguard body (component zone 120); a side region of the mouthguard inner body (component zone 110); and an opposite side region of the mouthguard inner body from the second component zone (component zone 130). The frontal region is located on an opposite side of a teeth-receiving protective channel to the side region and opposite side region. In this example the frontal region is located on an inner side of the body relative to the protective channel, and the side region and opposite side regions are located on an outer side of the body relative to the protective channel. Outer body member cover 160 is mounted to the body thereby to seal components mounted on both the outer side of the inner body relative to the protective channel thereby to cover and the inner side of the inner body relative to the protective channel.

It will be appreciated that the protective mouthguard of FIG. 1A to FIG. 1D includes a mouthguard body which is defined by a frontal region which protects the subject's incisors, and two opposed side regions which respectively protect oppose side molars. The mouthguard body is defined by a curved channel having: an inner wall, which abuts an inner side of the subject's teeth; an outer wall, which abuts an outer side of the subject's teeth; and a bridge, which connects between respective lower edges of the inner wall and the outer wall. In these examples, the one or more light emitting components and one or more light sensor components are embedded in one of the side regions. The individual examples are described below:
- In the example of FIG. 1A, a PCB component zone 150 is embedded in the body, and includes pair of light emitting components in the form of LEDs 151 and 153. LEDs 151 and 153 are embedded in the body within the outer wall, and configured to emit light at the controlled wavelength externally of the body in an outward direction, thereby to, in use, cause the emitted light to travel into cheek area body tissue of the subject. A light sensing component, in the form of a photodiode 152, is also embedded in the body within the outer wall within component zone 150. This photodiode is configured to in use sense light emitted by LEDs 151 and 153, with that light being affected by interaction with the cheek region body tissue of the subject. It will be appreciated that LEDs 151 and 153 in this example preferably emit a light having a wavelength in the green band. In this example, component zone 150 is positioned such that LEDs 151 and 153 and photodiode 152 have respective lens components which present externally of the mouthguard body, which otherwise has an opaque outer layer.

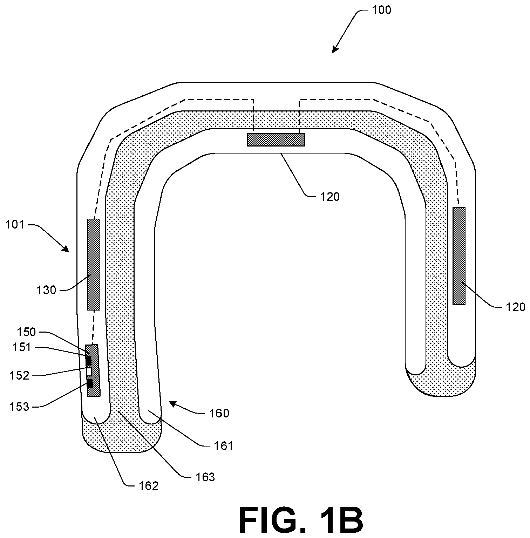

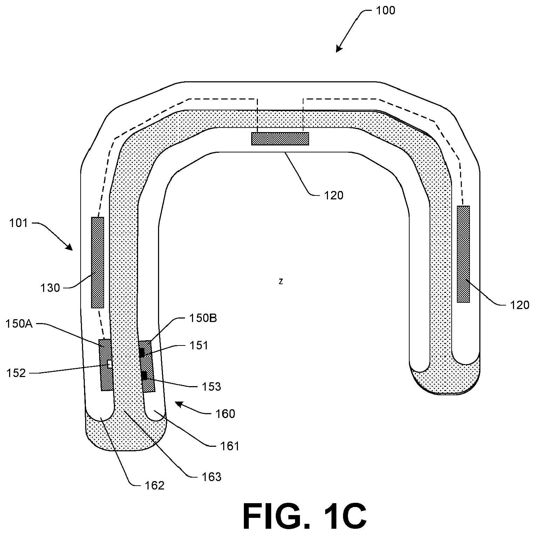

- In the example of FIG. 1B, a PCB component zone 150 is embedded in the body, and includes pair of light emitting components in the form of LEDs 151 and 153. LEDs 151 and 153 are embedded in the body within the outer wall, and configured to emit light at the controlled wavelength externally of the body in an outward direction, thereby to, in use, cause the emitted light to travel into cheek area body tissue of the subject. A light sensing component, in the form of a photodiode 152, is also embedded in the body within the outer wall within component zone 150. This photodiode is configured to in use sense light emitted by LEDs 151 and 153, with that light being affected by interaction with the cheek region body tissue of the subject. It will be appreciated that LEDs 151 and 153 in this example preferably emit a light having a wavelength in the green band. In this example, component zone 150 is positioned such that LEDs 151 and 153 and photodiode 152 have respective lens components which are embedded within the mouthguard body, with transparent windows overlying the lens components through the outer layer of the mouthguard body, which is otherwise an opaque outer layer.
- In the example of FIG. 1C, PCB component zones 150A and 150B is embedded in the body. These provide pair of light emitting components in the form of LEDs 151 and 153. LEDs 151 and 153 are embedded in the body within the inner wall, and configured to emit light at the controlled wavelength externally of the body in an outward direction, thereby to, in use, cause the emitted light to travel into gum area body tissue of the subject. A light sensing component, in the form of a photodiode 152, is also embedded in the body within the outer wall within component zone 150B. This photodiode is configured to in use sense light emitted by LEDs 151 and 153, with that light being affected by interaction with the hum region body tissue of the subject. It will be appreciated that LEDs 151 and 153 in this example preferably emit a light having a wavelength in the red band. In this example, component zone 150 is positioned such that LEDs 151 and 153 and photodiode 152 have respective lens components which present externally of the mouthguard body, which otherwise has an opaque outer layer.
- In the example of FIG. 1C, PCB component zones 150A and 150B is embedded in the body. These provide pair of light emitting components in the form of LEDs 151 and 153. LEDs 151 and 153 are embedded in the body within the inner wall, and configured to emit light at the controlled wavelength externally of the body in an outward direction, thereby to, in use, cause the emitted light to travel into gum area body tissue of the subject. A light sensing component, in the form of a photodiode 152, is also embedded in the body within the outer wall within component zone 150B. This photodiode is configured to in use sense light emitted by LEDs 151 and 153, with that light being affected by interaction with the hum region body tissue of the subject. It will be appreciated that LEDs 151 and 153 in this example preferably emit a light having a wavelength in the red band. In this example, component zones 150A and 150B are positioned such that LEDs 151 and 153 and photodiode 152 have respective lens components which are embedded within the mouthguard body, with transparent windows overlying the lens components through the outer layer of the mouthguard body, which is otherwise an opaque outer layer.

In other embodiments the LED and/or photodiode are not mounted to the same PCB as other components, and are instead cabled/wired.

In terms of construction thereby to allow the LEDs and photodiode to interact with the environment beyond the outer surface of the mouthguard body, some embodiments make use of a cutting technique thereby to expose the respective lenses. This may include a current technique which exposes substantially all of the component zone(s) providing LEDs/photodiodes, or which exposes just the component lenses. An example technique for forming a transparent window in the outer mouthguard body layer includes:
- Forming a base layer using a dentition mould. Where the device is configured for bodily uses other than a mouthguard, an alternate mould shape is used.
- Affixing PCB components to the base layer.
- Optionally applying one or more protective intermediate layers overlying the PCB components.
- Placing stencils over regions which are to be exposed, for example LEDS and photodiodes. These stencils may be hollow domes which overlie the areas which are to be exposed. They are preferably held in place using hot glue. A given stencil may be used to cover a dingle LED or photodiode, or a region of the PCB containing more than one LED and/or photodiode.
- Thermoforming an outer layer to the inner layer, thereby to seal in the PCB components.
- Following thermoforming of the outer layer, cutting away at hollow bulbous regions caused by the stencils. Preferably, the cutting operation is performed thereby to cut into the stencil (cutting in the approximate plane of the outer layer), as opposed to cutting around the stencil (i.e. cutting normally to the approximate plane of the outer layer).
- Once the cutting is performed, peeling away the hot glue that was used to stick the stencil to the PCB, thereby to form a window. Preferably the process includes smoothing the edges of the formed window with a hot scalpel, lathe machine or similar. Assuming the stencil was appropriately applied, the relevant PCB components will be visible through the window. Additional trimming may be required if the stencil was not correctly applied.
- Applying EVA or a similar material to seal the window, thereby providing a clear protective coating to the exposed PCB components. It is important to minimise imperfections such as bubbles in the EVA, as these may alter the performance of the PCB components.

In some embodiments an additional biocompatible material layer is applied over optical components. In some embodiments lenses, light pipes, and/or other such components are used to adjust focal properties of the optical components. In some embodiments one or more of the optical components form part of an in-mouth detection system (for example the photodiode may sense presence/absence of ambient light, and from that determine whether the mouthguard is being worn, or lack of reflection of LED light off the interior of the cheek may indicate that the mouthguard is out-of-mouth).

In a further embodiment, the mouthguard includes a first segment which is mounted inside the subject's mouth, and a second segment which is mounted outside the subject's mouth. This is configured such that at least one of the light emitting component passes light from one of the first or second segment to the other segment (e.g. through the cheek), where it is received by at least one of the light sensor component's.

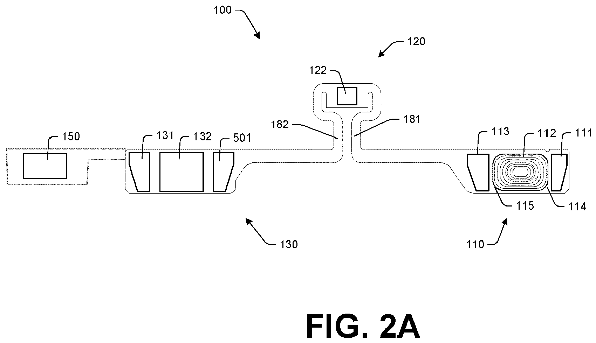

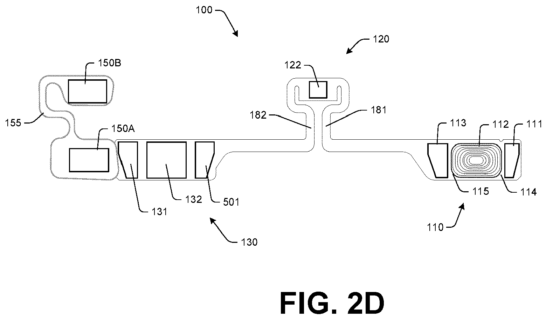

FIG. 2A to FIG. 2D illustrate example instrumented components 101 according to embodiments. These are configured for mounting in a mouthguard body thereby to provide an instrumented mouthguard, for example as described above in connection with FIG. 1A to FIG. 1D.

As shown in FIG. 2A to FIG. 2D, component 101 is defined by a flexible circuit board substrate which is configured such that one or more conductive members electronically couples component zones (e.g. printed circuit board regions). The flexible circuit board in this manner defines a conductive member which is irregularly shaped such that it is configured to enable fitting of the component zones at desired locations on mouthguard bodies of varied shapes and sizes. More particularly, a PCB is formed to meander between component zones in a manner that allows for customisable fitting, whilst providing for added flexibility and robustness when the mouthguard is used. This presents a significant advantage over non-meandering PCBs, or the use of wires interconnecting distinct PCBs.

The PCB substrate illustrated in FIG. 2A to FIG. 2D may be of variable thickness, and/or have rigidity supports applied, thereby to adjust rigidity on a special basis thereby to protect PCB components as required for robustness.

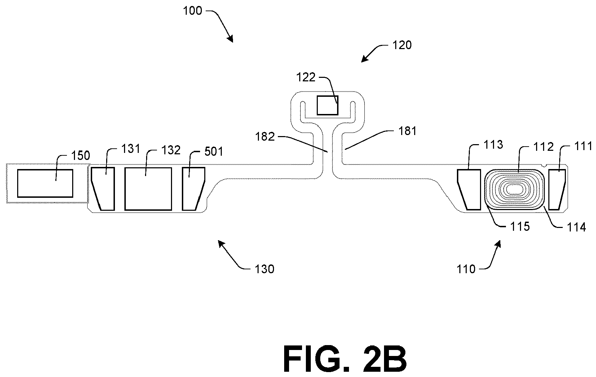

Component 101 includes three component zones:
- A right side component zone 110. In some implementations the right side component zone is configured to support PCB components including an accelerometer(3-axis), wireless communications unit, memory and microprocessor.
- A frontal component zone 120. In some implementations, component zone 120 is split provides an accelerometer supporting zone configured to be positioned on the outer side of the front teeth (for a 3-axis accelerometer). In some embodiments the frontal zone additionally includes a low-G accelerometer and/or a gyroscope.
- A left side component zone 130. In some implementations the left side component zone provides mounting locations for an accelerometer (3-axis), battery charging unit, and a battery mounting location.
- The positioning of components described above, and shown in FIG. 2B, is an example only, and in other embodiments alternate configurations of components are distributed between the component zones.

A flexible connector member, defined by part of the PCB substrate onto which conductors connects these zones, has a first segment 181 which electronically couples right size component zone 110 and frontal component zone 120, and a second segment 182 which electronically couples front component zone 120 and left side component zone 130. As shown in FIG. 2A TO FIG. 2D, these segments are meandering. In this example, as with examples above, the meandering is such that, segment 181 is greater than the length of the separation of connection points with zones 110 and 120, and segment 182 is greater than the separation of connection points with zones 120 and 130.

The flexible connector member provides a flexible substrate onto which conductive strips and a plurality of PCB components are mounted (for example PCB components in zones 110, 120 and 130). In some embodiments the flexible substrate has an increased thickness in certain regions thereby to provide increased rigidity for PCB components that are susceptible to damage as a result of PCB flexion (for example see regions 111, 112 and 113 discussed below). In some embodiments additional materials are applied to the flexible substrate thereby to increase rigidity where required.

In the embodiment of FIG. 2A to FIG. 2D, zone 110 is defined by three substantially rigid PCB regions 111, 112 and 113, interconnected by comparatively flexible regions (flex connectors) 114 and 115. This enables a better fit of zone 110 to a curved surface; in the present embodiment it is configured to mounted in a right cheek region of the mouthguard body. Zone 110 includes a range of electronic components, including:
- A 3-axis accelerometer.
- A microprocessor (for example a Qualcomm CSR1012).
- A memory module (for example a Macronix MX25L3233).
- A wireless communications module, in this embodiment being a Bluetooth module coupled to a Bluetooth antenna (not shown), for example, an antenna configured to be mounted such that it runs across a frontal region of the mouthguard forward of a wearer's teeth.
- A coupling port to a programming tab (not shown).
- A Light-Emitting Diode configured to be visible through the mouthguard body (not shown), in order to provide a device state indication to a user. For example, this is configured to be positioned behind the wearer's top lip.

It should be appreciated that the variations in rigidity within zone 110 (and across the component generally) is selected based at least in part of PCB components that are to be mounted at the various locations. For example, in one embodiment one or more of regions 111, 112 and 113 is not rigid, thereby to allow improved curvature upon application to the mouthguard body, and PCB components mounted to the non-rigid region are selected and/or mounted in such a manner to remain robust in spite to flexion in the PCB substrate.

Zone 120 includes a PCB region 122 including a 3-axis accelerometer (which is configured to be mounted to the mouthguard body in a location that in use is positioned behind front teeth). In the present embodiment PCB region 122 additionally includes a gyroscope, and a second accelerometer which is configured for lower levels of acceleration. Specifically, each component zone includes a 3-axis high-G accelerometer, and one component zone additionally includes a low-G accelerometer.

Zone 130 is configured to be mounted on a left cheek region of the mouthguard body, and includes a PCB that carries a 3-axis accelerometer 131, along with a charging coil 132 to enable wireless charging of a battery unit 151.

In other implementations the battery unit is located in zone 110 or zone 120. In further embodiments additional components including the likes of gyroscopes may also be present at one or more of the component zones (for example a gyroscope in combination with an accelerometer at each component zone.

Segment 181 of the conductive member is configured such that, upon mounting to the mouthguard body, it traverses across a bottom region of the mouthguard body at a region approximately adjacent cuspid and first bicuspid (or, alternately, first and second teeth). This allows zone 120 to be provided on an internal region (behind teeth) and zone 110 provided on an external region (in front of teeth). A sealing cover is mounted to the body thereby to seal components mounted on both the outer side of the body relative to the protective channel thereby to cover and the inner side of the body relative to the protective channel.

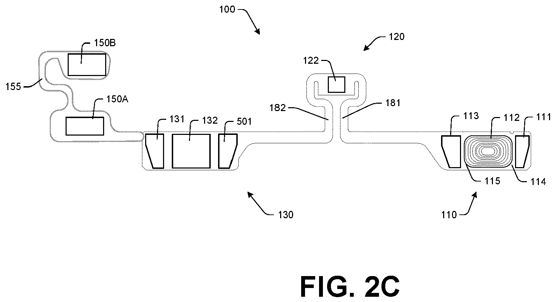

Each of FIG. 2A to FIG. 2D illustrate components configured to provide a pulse oximeter. These examples are discussed below.
- In the example of FIG. 2A, a component zone 150 is positioned on a region of the PCB which is separated from zone 130 by a short strip. Component zone 150 provides both LED and photodiode components, and optionally a microprocessor component configured to operate the LED and photodiode components thereby to provide pulse oximeter functionality. It will be appreciated that the photodiode and LED components are directed in a common direction, for example such that they aim outwards into the cheek (or optionally inwards into the gum)
- In the example of FIG. 2B, a component zone 150 is positioned on a region of the PCB which is in effect an extension of zone 130. Component zone 150 provides both LED and photodiode components, and optionally a microprocessor component configured to operate the LED and photodiode components thereby to provide pulse oximeter functionality. It will be appreciated that the photodiode and LED components are directed in a common direction, for example such that they aim outwards into the cheek (or optionally inwards into the gum)
- In the example of FIG. 2C, a component zone 150A is positioned on a region of the PCB which is separated from zone 130 by a short strip. A component zone 150B is positioned on a region of the PCB which is separate from zone 150A, preferably by a meandering path 155 (similar to region 181), thereby to allow some flexibility in the positioning of zone 150B relative to zone 150B, as these must be opposed across the subjects gum region. One of zone 150A and 150B provides one or more LEDs, with the other providing one or more photodiodes. The LED(s) and photodiode(s) aim inwards towards each other. Optionally, one of these provides a microprocessor component configured to operate the LED and photodiode components thereby to provide pulse oximeter functionality.
- In the example of FIG. 2C, a component zone 150A is positioned on a region of the PCB which is an extension of zone 130 by. A component zone 150B is positioned on a region of the PCB which is separate from zone 150A, preferably by a meandering path 155 (similar to region 181), thereby to allow some flexibility in the positioning of zone 150B relative to zone 150B, as these must be opposed across the subjects gum region. One of zone 150A and 150B provides one or more LEDs, with the other providing one or more photodiodes. The LED(s) and photodiode(s) aim inwards towards each other. Optionally, one of these provides a microprocessor component configured to operate the LED and photodiode components thereby to provide pulse oximeter functionality.

In each of these examples, data collected via operation of the LEDs and photodiodes to provide pulse oximeter functionality is able to be stored in the memory module of zone 101, and/or communicated externally of the device via wireless communication means (also provided in this example in zone 101).

It will be appreciated that the pulse oximeter components could in further embodiments be provided adjacent zone 101 as opposed to adjacent zone 130.

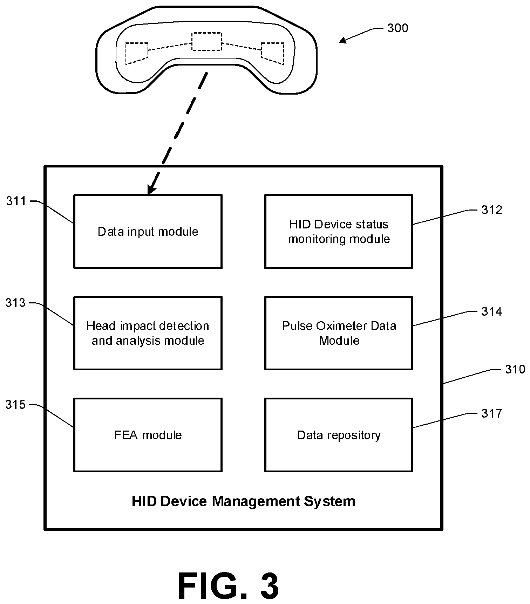

FIG. 3 illustrates an example technology framework, configured to enable monitoring of head impacts and physical performance for one or more subjects in a sporting activity.

The framework is described by reference to a head impact detection (HID) device, in the form of an instrumented mouthguard 300, and an HID Device Management System 310, which takes the form of a computing device (for example a PC, notebook, tablet or smartphone) or a plurality of computing devices (for example various processing functionalities may be performed by cloud-hosted components). Instrumented mouthguard 300 includes a microprocessor configured to execute onboard software instructions, and it will be appreciated that various functions described as being performed by system 310 may in further embodiments be performed in whole or in part by mouthguard 300.

Software is described herein by reference to various modules. The term "module" refers to a software component that is logically separable (a computer program), or a hardware component. The module of the embodiment refers to not only a module in the computer program but also a module in a hardware configuration. The discussion of the embodiment also serves as the discussion of computer programs for causing the modules to function (including a program that causes a computer to execute each step, a program that causes the computer to function as means, and a program that causes the computer to implement each function), and as the discussion of a system and a method. For convenience of explanation, the phrases "stores information," "causes information to be stored," and other phrases equivalent thereto are used. If the embodiment is a computer program, these phrases are intended to express "causes a memory device to store information" or "controls a memory device to cause the memory device to store information." The modules may correspond to the functions in a one-to-one correspondence. In a software implementation, one module may form one program or multiple modules may form one program. One module may form multiple programs. Multiple modules may be executed by a single computer. A single module may be executed by multiple computers in a distributed environment or a parallel environment. One module may include another module. In the discussion that follows, the term "connection" refers to not only a physical connection but also a logical connection (such as an exchange of data, instructions, and data reference relationship). The term "predetermined" means that something is decided in advance of a process of interest. The term "predetermined" is thus intended to refer to something that is decided in advance of a process of interest in the embodiment. Even after a process in the embodiment has started, the term "predetermined" refers to something that is decided in advance of a process of interest depending on a condition or a status of the embodiment at the present point of time or depending on a condition or status heretofore continuing down to the present point of time. If "predetermined values" are plural, the predetermined values may be different from each other, or two or more of the predetermined values (including all the values) may be equal to each other. A statement that "if A, B is to be performed" is intended to mean "that it is determined whether something is A, and that if something is determined as A, an action B is to be carried out". The statement becomes meaningless if the determination as to whether something is A is not performed.

The term "system" refers to an arrangement where multiple computers, hardware configurations, and devices are interconnected via a communication network (including a one-to-one communication connection). The term "system", and the term "device", also refer to an arrangement that includes a single computer, a hardware configuration, and a device. The system does not include a social system that is a social "arrangement" formulated by humans.

At each process performed by a module, or at one of the processes performed by a module, information as a process target is read from a memory device, the information is then processed, and the process results are written onto the memory device. A description related to the reading of the information from the memory device prior to the process and the writing of the processed information onto the memory device subsequent to the process may be omitted as appropriate. The memory devices may include a hard disk, a random-access memory (RAM), an external storage medium, a memory device connected via a communication network, and a ledger within a CPU (Central Processing Unit).

In the example of FIG. 4, instrumented mouthguard 300 communicates with system 310 via a wireless connection. This may include a range of wireless technologies, including WiFi, Bluetooth, and/or other radio bands. In some embodiments communications between mouthguard 300 and system 310 progresses via one or more intermediate devices, including on-body retransmitting devices, devices in mesh networks, routers, and so on.

Data transmitted by mouthguard 300 is received by a data input module 311. Data input module 311 is configured to extract and sort input data, thereby to organise that data into memory accessible to system 310 (for example in one or more databases). This includes identifying a unique device identifier associated with mouthguard 300, which is preferably associated with a unique human subject. The data may include, for example, any one or more of: (i) a time-series of sensor readings, with associated time correlation data (such as a timestamp at the commencement of the series, and a known sampling rate); (ii) data packets representative of identified potential impact events (for example where the mouthguard is configured to operate in at least one setting where it transmits sensor data only where that sensor data has threshold values which indicate a potential impact); and (iii) output data from an onboard processing module (for example on onboard FEA module which provides an output based on a dosage input, the dosage input being derived from sensor data); and (iv) regular beacon/heartbeat data packets representative of device status. Other data may also be received, for example physiological data (such as heart rate, breathing rate, etc).

Data received and processed via input module 311 is stored in a data repository 317, where it is available for accessing and processing by other modules of system 310.

A HID device status monitoring module 312 is configured to process data received via input module 311 thereby to determine a current status of mouthguard 300 and optionally one or more further mouthguard devices. This may be used to assess whether one or more mouthguards are in a fault state or the like. In some embodiments module 312 is configured to enable two-way communication with mouthguard 300, for example to enable remote switching of mouthguard 300 between multiple distinct operational settings (for example one optimised for impact detection, and one optimised for physical performance assessment).

A head impact detection and analysis module 313 is configured to process data derived from sensors of mouthguard 300 thereby to provide metrics representative of severity of an observed impact event, for example via an event recoding protocol which dynamically adjusts event duration as described further above. It will be appreciated that there are a range of technologies which may be used for this processing, for example using techniques to process linear and/or rotational acceleration, optionally using AI methods and/or benchmarking against existing data. In this example, module 313 operates in conjunction with a Finite Element Analysis (FEA) module 315. Module 313 is configured to process sensor data thereby to define a dosage input signal. This may include processing time correlated data from multiple sensors thereby to determine an acceleration value at a defined location (for example at the centre of gravity of the subject's head, preferably based on transforms which are individually customised for the particular human subject based on their mouthguard and physical head configuration). This acceleration value is passed to FEA module 315, which performs analysis thereby to provide one or more metrics representative of predicted effect of the acceleration to the subject's brain (impact parameters), thereby to provide data which assists in understanding anticipated severity of a head impact.

A pulse oximeter data module 314 is configured to control operation and data collection for pulse oximeter components, for example as described further above. This may include triggering operation, batching data, and the like.

It will be appreciated that the disclosure above provides for collecting human physiological data from a protective mouthguard.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

The corresponding structures, materials, acts, and equivalents of all means or step plus function elements, if any, in the claims below are intended to include any structure, material, or act for performing the function in combination with other claimed elements as specifically claimed. The description of the present invention has been presented for purposes of illustration and description, but is not intended to be exhaustive or limited to the invention in the form disclosed. Many modifications and variations will be apparent to those of ordinary skill in the art without departing from the scope and spirit of the invention. The embodiment was chosen and described in order to best explain the principles of the invention and the practical application, and to enable others of ordinary skill in the art to understand the invention for various embodiments with various modifications as are suited to the particular use contemplated.

Various aspects of the present disclosure may be embodied as a program, software, or computer instructions embodied in a computer or machine usable or readable medium, which causes the computer or machine to perform the steps of the method when executed on the computer, processor, and/or machine. A program storage device readable by a machine, tangibly embodying a program of instructions executable by the machine to perform various functionalities and methods described in the present disclosure is also provided.

A system and method of the present disclosure may be implemented and run on a general-purpose computer or special-purpose computer system. The terms "computer system" and "computer network" as may be used in the present application may include a variety of combinations of fixed and/or portable computer hardware, software, peripherals, and storage devices. The computer system may include a plurality of individual components that are networked or otherwise linked to perform collaboratively, or may include one or more stand-alone components. The hardware and software components of the computer system of the present application may include and may be included within fixed and portable devices such as desktop, laptop, and/or server. A module may be a component of a device, software, program, or system that implements some "functionality", which can be embodied as software, hardware, firmware, electronic circuitry, or etc.

Although specific embodiments of the present invention have been described, it will be understood by those of skill in the art that there are other embodiments that are equivalent to the described embodiments. Accordingly, it is to be understood that the invention is not to be limited by the specific illustrated embodiments, but only by the scope of the appended claims.

It should be appreciated that in the above description of exemplary embodiments of the invention, various features of the invention are sometimes grouped together in a single embodiment, FIG., or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the Detailed Description are hereby expressly incorporated into this Detailed Description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those skilled in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

Similarly, it is to be noticed that the term coupled, when used in the claims, should not be interpreted as being limited to direct connections only. The terms "coupled" and "connected," along with their derivatives, may be used. It should be understood that these terms are not intended as synonyms for each other. Thus, the scope of the expression a device A coupled to a device B should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

Thus, while there has been described what are believed to be the preferred embodiments of the invention, those skilled in the art will recognize that other and further modifications may be made thereto without departing from the spirit of the invention, and it is intended to claim all such changes and modifications as falling within the scope of the invention. For example, any formulas given above are merely representative of procedures that may be used. Functionality may be added or deleted from the block diagrams and operations may be interchanged among functional blocks. Steps may be added or deleted to methods described within the scope of the present invention.

## Claims

1. A method for recording events detected by an instrumented mouthguard device, the method including:
(A) collecting data from a plurality of instrumented sensor components provided by the instrumented mouthguard device;
(B) processing at least a subset of the data collected at (A) thereby to identify presence of an over-threshold condition;
(C) in the case that an over-threshold condition is identified:
(i) commencing recording of event data from a start point preceding presence of the over-threshold condition by a predefined leading period; and
(ii) determining a point in time where an over-threshold condition is no longer detected; and
(iii) ending recording of the event data at an end point following the point in time where an over-threshold condition is no longer detected by a predefined trailing period;
such that, for each event, a duration of time for which event data is recorded is dynamically set responsive to the length of time for which the identified over-threshold condition persists.

2. A method according to claim 1 wherein the over-threshold condition is defined by reference to a greater-than-threshold acceleration value being detected by at least one sensor of the instrumented mouthguard device.

3. A method according to claim 1 or claim 2 wherein the over-threshold condition is defined by reference to a greater-than-threshold acceleration value being detected by at least two sensors of the instrumented mouthguard device.

4. A method according to claim 2 or claim 3 wherein the greater-than-threshold acceleration value includes a linear acceleration value and/or rotational acceleration value.

5. A method according to any preceding claim wherein the over-threshold condition is defined by reference to a greater-than-threshold rotational velocity value being detected by at least one sensor of the instrumented mouthguard device.

6. A method according to any preceding claim wherein the instrumented mouthguard device includes at least one accelerometer and at least one gyroscope, and wherein the over-threshold condition is defined by reference to data collected by the gyroscope.

7. A method according to any preceding claim wherein the instrumented mouthguard device includes a processing unit which receives a data feed from the plurality of sensor components, wherein the processing unit executes software instructions which implement steps (A) to (C).

8. A method according to any preceding claim wherein there are multiple peak acceleration spikes between the end of the leading period and the start of the trailing period.

9. An instrumented mouthguard device including:
a mouthguard body configured to be inserted into the mouth of a user;
plurality of instrumented sensor components embedded in the body;
a processing unit embedded in the body; and
a memory module embedded in the body;
wherein the processing unit is configured to receive data from the plurality of instrumented sensor components, and process that data thereby to cause recording discrete sets of event data in the memory module based on an event recording protocol, wherein the event recording protocol includes:
(i) processing at least a subset of the data collected by the instrumented sensor components thereby to identify presence of an over-threshold condition;
(ii) in the case that an over-threshold condition is identified, commencing recording of event data from a start point preceding presence of the over-threshold condition by a predefined leading period to an end point following the point in time where the over-threshold condition is no longer detected by a predefined trailing period;
such that, for each event, a period of time for which event data is recorded is dynamically set responsive to the length of time for which the identified over-threshold condition persists.

10. An instrumented mouthguard according to claim 9, wherein the over-threshold condition is defined by reference to a greater-than-threshold acceleration value being detected by at least one sensor of the instrumented mouthguard device.

11. An instrumented mouthguard according to claim 9 or claim 10, wherein the over-threshold condition is defined by reference to a greater-than-threshold acceleration value being detected by at least two sensors of the instrumented mouthguard device.

12. An instrumented mouthguard according to claim 10 or claim 11 wherein the greater-than-threshold acceleration value includes a linear acceleration value and/or rotational acceleration value.

13. An instrumented mouthguard according to any one of clams 9 to 12, wherein the over-threshold condition is defined by reference to a greater-than-threshold rotational velocity value being detected by at least one sensor of the instrumented mouthguard device.

14. An instrumented mouthguard according to any one of clams 9 to 13, wherein the instrumented mouthguard device includes at least one accelerometer and at least one gyroscope, and wherein the over-threshold condition is defined by reference to data collected by the gyroscope.

15. An instrumented mouthguard according to any one of clams 9 to 14, wherein there are multiple peak acceleration spikes between the end of the leading period and the start of the trailing period.
